(19) Europäisches Patentamt
European Patent Office
Office européen des brevets

(11) Publication number: **0 460 311 A1**

# (12) EUROPEAN PATENT APPLICATION

(21) Application number: **90306145.5**

(22) Date of filing: **06.06.90**

(51) Int. Cl.5: **C07C 41/58**, C07C 43/04, B01J 20/12

(43) Date of publication of application: **11.12.91 Bulletin 91/50**

(84) Designated Contracting States: **AT BE CH DE DK ES FR GB IT LI LU NL SE**

(71) Applicant: **E.I. DU PONT DE NEMOURS AND COMPANY**
**1007 Market Street**
**Wilmington Delaware 19898(US)**

(72) Inventor: **Dunson, James B.**
**202 Winslow Road**
**Newark, Delaware 19711(US)**
Inventor: **Mc Cartney, Robert F.**
**525 Country Club Drive, Woodbrook**
**Wilmington, Delaware 19803(US)**
Inventor: **Warwas, Edwin J.**
**209 N. Pembrey Drive, Pembrey**
**Wilmington, Delaware 19803(US)**

(74) Representative: **Woodcraft, David Charles et al**
**BROOKES & MARTIN High Holborn House**
**52/54 High Holborn**
**London, WC1V 6SE(GB)**

## (54) A method for removing odorous impurities from dimethyl ether.

(57) A method is described for removing odorous impurities from dimethyl ether which comprises contacting the dimethyl ether with an acidic clay, such as a montmorillonite clay. The dimethyl ether is conveniently pumped through a bed of the acidic clay in such a way that the contact time for treatment is about 10 to 20 seconds.

EP 0 460 311 A1

The present invention concerns a method for preparing dimethyl ether free of odorous or odor-forming impurities.

Dimethyl ether was originally obtained as a minor by-product from the manufacture of synthetic methanol. As a result of improved synthetic processes for methanol, the amount of by-product dimethyl ether became sharply reduced. At the same time, an increased demand for dimethyl ether made it necessary to develop a new route to this material. This led to its manufacture by the catalytic dehydration of methanol, as follows:-

$$2CH_3OH \text{ ---> } CH_3OCH_3 + H_2O$$

The increased demand for dimethyl ether resulted from its use in a wide variety of industrial applications such as a solvent, extraction agent and aerosol propellant. This latter application has been growing rapidly in recent years because most of the other commonly used aerosol propellants cause damage to the ozone layer or promote smog formation at ground level. For many of these uses, especially as an aerosol propellant, it is very important that the dimethyl ether be completely odor free.

Numerous catalysts and processes for the synthesis of dimethyl ether are disclosed in the patent literature. For example, in U.S. Patent No. 4,605,788, it is disclosed that an aluminosilicate catalyst containing about 94% alumina and 6% silica can be effectively used to dehydrate methanol, yielding a product with about 57% dimethyl ether, 20% methanol and 23% water. Usually the dimethyl ether is then separated by distillation, and the unreacted methanol returned to the reaction. Several such distillation procedures are disclosed in U.S. Patent No. 4,802,956, wherein the difficulties in removing trace quantities of impurities such as methyl formate, hydrocarbons, amines and sulfides by distillation are disclosed. This patent proposes a highly specific method of distillation which enables the use of a single column to avoid the expense of two columns. One of the advantages claimed for this specific procedure is the production of an odorless dimethyl ether.

Unfortunately the process of distillation does not always make it possible to obtain an odor-free product, even when repeated on a commercially pure dimethyl ether using carefully controlled conditions designed to eliminate odorous materials. This is partly because only trace quantities of certain impurities (e.g., at part-per-million level) are sufficient to cause the dimethyl ether to be unsuitable for certain demanding applications. The problem is further complicated because many of these odor-formers may vary in amount from day to day depending on the source of raw materials, slight changes in catalyst activity or processing conditions, contamination by nearby operations, or by entirely unknown causes. Many specific trace impurities are transient and not chemically identified in time to take corrective action. A method of purification may be effective one day and ineffective the next because the nature of the impurity or its amount has changed.

One method to handle this problem has been to carefully check each production lot of dimethyl ether and select those lots for aerosol applications which appear to be odor-free. In using this method, there may be insufficient odor-free dimethyl ether on hand to supply urgent needs for aerosol applications. There is also the risk that odor will develop later in these lots on standing or when mixed with other ingredients in an aerosol spray. These problems have been a significant deterrent to developing a large market for dimethyl ether in this and similarly demanding applications requiring odor-free material.

The technique of absorbing or otherwise trapping such trace impurities using a solid absorbent or molecular sieve has sometimes been used to solve similar problems with other products. However it is usually not possible to predict whether a particular absorbent will remove the troublesome impurities from a specific product. This is because the mechanism of absorption depends on such factors as the pore size distribution of the absorbent or molecular sieve, the amount and character of the surface area, the nature of its surface charges, the history of the absorbent, the temperature and contact time required for the treatment, and the nature of the impurities being removed compared to the nature of the product being purified. Under these conditions the selection of an absorbent and necessary treatment conditions to solve a particular contamination problem is largely a matter of trial and error. Following these experiments, repeated trials on different lots of the product are required to make sure the solution is a permanent one. At present, a cheap and reliable method using simple absorption for removing odorous or odor-forming impurities from dimethyl ether to solve the previously described production and quality problems is not readily available.

Methods Used to Solve the Aforementioned Problem

The goal of the present invention is to present a method for removing odorous or odor-forming impurities from dimethyl ether. That is, the present invention presents a method for treating dimethyl ether with an acidic montmorillonite clay which produces an odor-free dimethyl ether suitable for applications where the presence of an odor would be undesirable.

According to the present invention, when production lots of commercially pure dimethyl ether were found unsuitable for use in aerosols, initial attempts to solve the problem by careful redistillation techniques to remove the odorous impurities proved unsuccessful. Further attempts to remove the odorous impurities using absorption techniques were carried out by treating the dimethyl ether with a wide variety of typical absorbent materials such as charcoal, alumina, silica gel, diatomaceous earth, fuller's earth, zeolite, ion exchange resins, attapulgite clay and other common absorbents. None of these treatments was effective. However, upon trying the material commercially sold to homes for use in absorbing the odor from the urine of household pets, particularly the material used in litter boxes for cats as an absorbant, this material appeared to solve the odor problem in dimethyl ether. Further investigation showed that this material, identified as an acidic montmorillonite clay, was satisfactory in lot after lot of dimethyl ether production. Attempts identify the source of the odor problem, and determine what impurities were being removed by the above treatment with acidic clay were only partially successful. It has been found that production lots of dimethyl ether which were high in methylal (dimethoxy methane) were also unsatisfactory in odor, and that lots which were free of methylal were satisfactory in odor. Further, it has been found that dimethyl ether to which we had added very small quantities of methylal were effectively cleansed of this impurity by the above clay treatment. Nevertheless the methylal itself did not appear to be the major odor-former, but instead appears to be formed at the same time as the odorous impurities. Apparently these other impurities were present in too low a concentration to show up in the analysis used. The methylal appears to be a symptom of odor problems rather than the direct source. However, the treatment of dimethyl ether with acidic clay was effective against both the methylal and other causes of the odor problems.

The following features are important in carrying out this process. It is important that the dimethyl ether be fully contacted by the acidic montmorillonite clay. This can be done by mixing the clay and the dimethyl ether in an agitated vessel and filtering off the clay, but it is more convenient to simply pump the liquified dimethyl ether through a stationary bed of the clay. The design of this unit should be such as to minimize bypassing of the clay by the dimethyl ether. That is, the flow of dimethyl ether must be directed so that it passes through the entire bed of clay, thus insuring that all the dimethyl ether has properly treated. A cylinder loaded with the acidic clay has been found adequate, with the dimethyl ether fed in at one end and the treated material leaving the other through a perforated section lined with a 200 mesh screen. A cleanup filter is also desirable to remove any clay fines escaping from the treatment unit. Other eqipment designs which will provide the same contacting efficiency will be obvious to those skilled in the art. The flow through this unit should be controlled at a level to minimize physical degradation of the clay by attrition. Too high a flowrate would result in excessive carryover of clay fines to the cleanup filter and frequent shutdowns for cleaning it out or the installation of an unnecessarily large cleanup unit. Too low a flowrate would result in an increased size for the contacting unit. It is apparent that a wide range of flowrates can be used depending on the design and economics of the equipment chosen. It has been found that a satisfactory balance of equipment sizes can be obtained with a velocity of 0.5 to 1.5 cm per second through the filter screen being preferred.

The flow through the clay bed must also be regulated to provide adequate contacting time for the removal of the odorous impurities. This will, of course, partly depend on the level and type of impurities present in the dimethyl ether. Too short a time will result in inadequate odor removal; too long a time would require that the treatment equipment be of excessive size. A contact time of only 10 to 20 seconds is generally adequate and is therefore preferred.

Depending on the level of impurites in the dimethyl ether, the clay will eventually lose its effectiveness for odor removal. This should be checked by periodic sampling and checking of the treated dimethyl ether, and the clay replaced when it shows signs of approaching saturation. On experience indicates that 9 kilograms of clay will effectively treat more than 16000 kilograms of dimethyl ether before replacement is necessary. If the dimethyl ether has very low levels of odorous impurities the clay will last much longer and become essentially an insurer of satisfactory odor-free quality.

A particularly preferred clay useful in the present invention is "Filtrol" acidic clay XJ-8407P or "Filtrol" Grade 124. It has a measured capacity for removal of ammonia of 0.80 milliequivalents per gram, and for trimethyl amine of 0.25 milliequivalents per gram. Other similar grades of acidic montmorillonite clay are also satisfactory, but should be checked against typical production lots of dimethyl ether to determine probable useage.

The packing of the clay in the treatment cylinder should be as even as possible so that no voids are

present. This can be accomplished by gentle shaking of the cylinder during loading. The even packing of clay in the cylinder will ensure the maximum treatment for the dimethyl ether passing through the column.

The temperature and pressure of the dimethyl ether should be such that it is present as a liquid. Treatment at ambient temperature is most convenient and is therefore preferred.

## Working Examples

The following are working examples of this invention.

### Practical Example 1

A stainless steel cylinder 17 cm in diameter and with a total length of 63 cm was used in continuous operation to purify commercial grade dimethyl ether. The cylinder was perforated and backed up with a 200 mesh stainless steel screen. The cylinder was placed inside a 22 cm cylinder completely closed at the top and bottom except for piping connections, with the top gasketed so that all incoming fluids would be fed into the perforated cylinder. The perforated cylinder was then loaded with 9 kilograms of acidic montmorillonite clay. The dimethyl ether was pumped to the top of the inner cylinder at ambient temperature and withdrawn from the bottom of the outer cylinder. It then was fed to a polishing or cleanup filter to remove any clay fines, and sent to storage or the shipping center. The dimethyl ether was fed at a rate of 3550 kilogram per hour for a period of 4.5 hours. The dimethyl ether before treatment had a harsh, biting odor. After treatment it was judged odor-free.

### Practical Example 2

The above equipment of Practical Example 1 was modified by replacing the top 46 cm of the perforated cylinder with a solid-walled cylinder to help prevent any bypassing, leaving the perforated cylinder and screen on the lowest 17 cm. Using this design it was found that 9.5 kilogram of clay could satisfactorily treat up to 89000 kilogram of dimethyl ether with satisfactory results, using a flowrate of 2630 kilogram per hour. All the treated product was judged to be odor-free.

### Practical Example 3

Production lots of dimethyl ether which had been judged unsatisfactory due to odor was analyzed by gas chromatographic mass spectroscopy and found to contain significant amounts of methylal, methyl formate and various isomers of butene and pentene. Using this formulation, a fresh sample of dimethyl ether not containing any of these impurities was then spiked with 1.5 parts per million of methyl formate, 1.0 part per million of 1-pentene, 1.0 part per million of 2-pentene, 1.3 parts per million of methylal, and 1.5 parts per million of methyl acetate. Then a 30 cc steel cylinder was filled with "Filtrol" Catalyst Grade 124. The clay was heated to 200 C for 2 hours and allowed to cool to room temperature in a desiccator. The spiked dimethyl ether was passed through the clay and collected for analysis in three sequential samples. Nearly all the methylal was effectively removed by this treatment. The first sample showed no detectable methylal. The second and third samples showed about a 75% reduction in methylal concentration, with much smaller effects on the other added impurities. Analytical results on the initial and treated dimethyl ether were as follows, using a gas chromatographic method of analysis with a flame ionization detector.

| Ingredient | Retention Time | Initial Sample | Treated Samples No. 1 | No. 2 | No. 3 |
|---|---|---|---|---|---|
| 1-pentene | 19.6 sec | 0.016% | 0.016% | 0.013% | 0.013% |
| methyl formate | 19.6 sec | above | above | above | above |
| 2-pentene(cis) | 20.7 sec | 0.013% | 0.016% | 0.013% | 0.011% |
| 2-pentene(trans) | 21.3 sec | 0.005% | 0.006% | 0.005% | 0.005% |
| methylal | 22.9 sec | 0.012% | not det. | 0.003% | 0.003% |
| methanol | 25.4 sec | 0.016% | 0.012% | 0.006% | 0.006% |
| methyl acetate | 26.2 sec | 0.025% | 0.026% | 0.022% | 0.019% |

Effect of the Invention

As the practical examples illustrate, the effect of this invention is that by the method of this invention it is possible to produce odor-free dimethyl ether starting from commerically impure dimethyl ether and to do this reliably and economically.

## Claims

1. A method for removing odorous impurities from dimethyl ether, which is characterized by treating the dimethyl ether with an acidic clay.

2. A method for removing methylal from dimethyl ether, which is characterized by treating the dimethyl ether with an acidic clay.

3. A method as defined in claims 1 or 2, in which the acidic clay is an acid-treated clay from the montmorillonite group.

4. A method as defined in any of claims 1 to 3, in which the treatment is performed at a temperature of 0°C to 40°C.

5. A method as defined in any of claims 1 to 4, in which the dimethyl ether is contacted with the clay for a time of 10 to 20 seconds.

European Patent Office

# EUROPEAN SEARCH REPORT

Application Number

EP 90 30 6145

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | PATENT ABSTRACTS OF JAPAN vol. 11, no. 136 (C-419)(2583) 30 April 1987, & JP-A-61 275239 (SOGO YATSUKOU) 05 December 1986, * the whole document * | 1 | C07C41/58<br>C07C4304<br>C07C43/04<br>B01J20/12 |
| A | BE-A-680062 (VEREINIGTE GLANZSTOFFABRIKEN) * claim 1 * | 1 | |
| A | DE-A-2458171 (TANAKA GIKEN) * page 6, line 18 - page 6, line 27; claim 1 * | 1 | |
| A | FR-A-641580 (DELCO-LIGHT) * page 2, line 32 - page 2, line 54 * | 1 | |
| A | US-A-1819496 (W.S. BAYLIS) * page 1, column 1, line 1 - page 1, column 1, line 14 * | 1 | |

TECHNICAL FIELDS SEARCHED (Int. Cl.5 )

C07C
B01J

The present search report has been drawn up for all claims

| Place of search | Date of completion of the search | Examiner |
|---|---|---|
| BERLIN | 06 FEBRUARY 1991 | PROBERT,C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document